# EUROPEAN PATENT APPLICATION

(11) **EP 3 653 226 A1**
(43) Date of publication of application: **20.05.2020**
(21) Application number: 18206883.3
(22) Date of filing: 17.11.2018
(51) Int. Cl.: A61K 41/00, A61K 47/60, A61K 49/00, A61P 31/00, A61P 35/00, A61P 17/00, A61P 27/02, C09B 47/00, C07F 15/04, C07D 487/22

(54) **BACTERIOCHLORIN FOR MEDICAL USE AND FOR GENERATING SINGLET OXYGEN AND OTHER REACTIVE OXYGEN SPECIES AND PRODUCTION PROCESS**

(71) Applicant: Christian-Albrechts-Universität zu Kiel, 24118 Kiel (DE)
(72) Inventor: Herges, Rainer, 24118 Kiel (DE); Peters, Morten, 24118 Kiel (DE)
(74) Representative: Taruttis, Stefan Georg

(57) **Abstract**

The compound comprises the general structure of a bacteriochlorin and can be used as a medicament, e.g. in photodynamic therapy, or in a technical process for generating singlet oxygen from gaseous or dissolved oxygen. The compound generates singlet oxygen and is stable against oxidation, e.g. it is not quickly inactivated by the singlet oxygen that it generates under irradiation.

## Description

The present invention relates to a novel compound comprising the general structure of a bacteriochlorin. The compound can be for use as a medicament, e.g. for use in medical treatment, especially in a medical treatment using activation of the compound by irradiation with light, which is also referred to as photodynamic therapy. The medical treatment can be the treatment of solid tumours, of a skin disease, an infection, e.g. a bacterial, fungal or viral infection, of macular degeneration, wherein the infectious agent may be resistant to at least one antibiotic. The compound generates singlet oxygen and optionally other reactive oxygen species (ROS) from oxygen, which singlet oxygen and ROS are highly reactive and inactivate e.g. bacteria, viruses and mammalian cells, e.g. tissue.

The compound has the advantage that it is stable against oxidation, e.g. it is not quickly inactivated by the singlet oxygen that it generates under irradiation. Further, the compound is useful in a technical process for generating singlet oxygen and ROS, because irradiation with a wavelength in the near infrared range, especially a wavelength of 740 nm, activates the compound to generate singlet oxygen from gaseous or dissolved oxygen. The compound can be derivatized by various substituents to adjust its hydrophilicity, respectively lipophilicity. Specifically, the compound can be derivatized by substituents, which render the compound non-immunogenic.

Further, the invention relates to an embodiment in which the compound contains a complexed metal ion, which is selected from alkaline earth metals and transition metals e.g. ions of Mg, Pt, Pd, Ni. In the embodiment in which the complexed metal ion is Ni²⁺, the compound does not produce singlet oxygen under irradiation. In this embodiment, the compound forms an oxidation-stable dye.

Further, the invention relates to a technical process for producing singlet oxygen, wherein the compound is preferably immobilized on a carrier, e.g. by a covalent linkage to the carrier, contacting the compound with oxygen and irradiating the compound. The oxygen can be contained in a gas, e.g. air, or in a liquid, e.g. water.

The invention also provides a process for producing the compound. The production process has the advantage of optionally being purely synthetic, i.e. without the use of reactants of natural origin. The production process also has the advantage of requiring only moderate temperatures, e.g. below 70°C, e.g. at room temperature, and that the process does not use a metal-containing catalyst.

### State of the art

Padeliporphin, which is known for use in photodynamic therapy of cancer, produces singlet oxygen from oxygen present in tissue. Padeliporphin has the disadvantage to contain the heavy metal palladium and that, under irradiation, it is degraded by the singlet oxygen it produces.

Peters and Herges, Beilstein J. Org. Chem. 2017, 13, 2659-2662 describe a synthetic method for producing isobenzofuran.

### Object of the invention

The object of the invention is to provide alternative compounds, which are stable against oxidation, especially for use as a medicament in the medical treatment e.g. of cancer or in a technical process for producing singlet oxygen and optionally ROS, and which can be produced e.g. without use of OsO₄ and at temperatures of below 100°C, e.g. at below 70°C. Preferably, the compounds generate singlet oxygen from surrounding oxygen under irradiation. More preferably, the compounds shall have an absorption in the wavelength range of 680-900 nm, which is generally known as the range for which human tissue is transmissible.

### Description of the invention

The invention achieves the object by the features of the claims, especially by providing a compound comprising 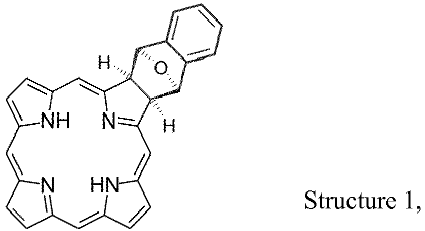 and/or preferably

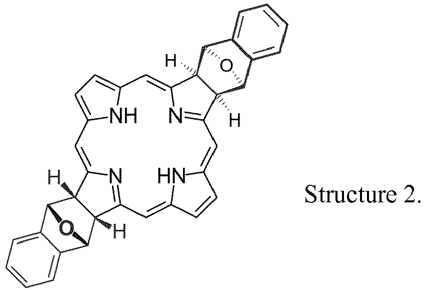

In a preferred embodiment, the compound comprises 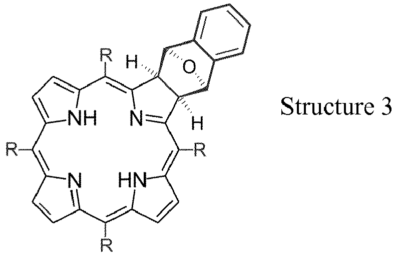 R, = H 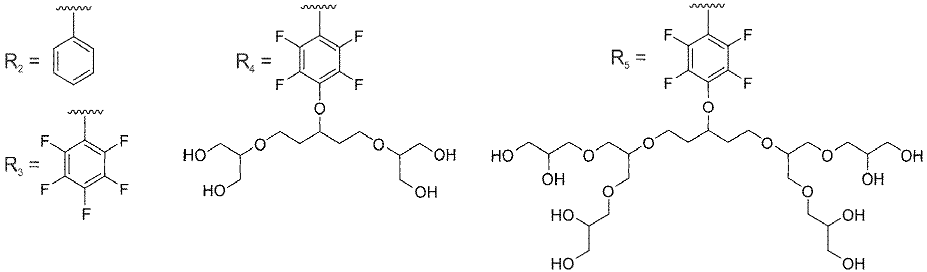 and/or, more preferred, 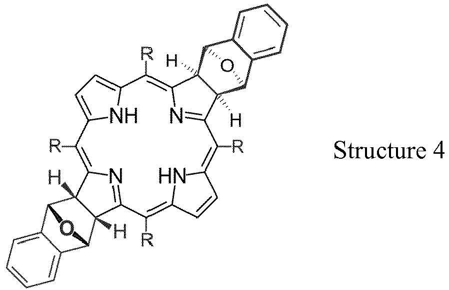 R₁ = H

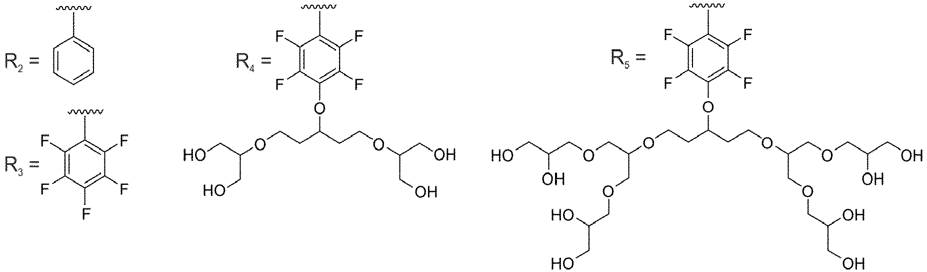

The substituents R1 to R5 shown here in the *meso* positions are examples for substituent R, which can be the same or a different substituent in each position. Generally, especially in Structure 1 and Structure 2, atoms having valences at which no substituent is explicitly shown, e.g. in the *β* positions, may be substituted with H or any other substituent.

The porphyrin moiety can optionally carry substituents, e.g. in at least one or all of the *meso* positions of the porphyrin moiety and/or in at least one or in all of the 8 *β* positions of the porphyrin ring. In Structure 3 and Structure 4 the substituents R can be different in each position or the same substituent. The substituents R can be selected from linear, branched or circular alkyl or aryl groups, e.g. C1 to C12, optionally containing at least one hetero atom, and aliphatic or aromatic groups, e.g. containing at least one five-membered or six-membered ring, optionally containing at least one hetero atom, and R can be as shown for Structure 4. Structure 3 and Structure 4 show R1 being H, R2 being phenyl, R3 being pentafluorophenyl, and R4 and R5 being para-substituted tetrafluorophenyl with the para-substituent being dendrimers of different lengths as preferred substituents R. Preferably, in Structure 3 and Structure 4, atoms having valences at which no substituent is explicitly shown, may be substituted with any substituent, preferably with H.

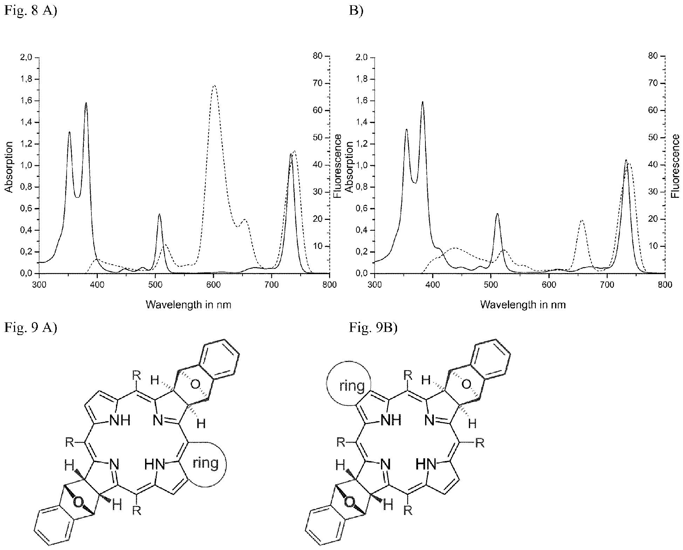

Generally, the carbon atoms in the 4, 5, 6 and 7 positions of the phenyl ring of the isobenzofuran moiety can be substituted with hydrogen or with any other substituent, e.g. a substituent selected from linear, branched or circular alkyl or aryl groups, e.g. C1 to C12, optionally containing at least one hetero atom, and aliphatic or aromatic groups, e.g. containing at least one five-membered or six-membered ring, which can be annelated to the phenyl ring of the isobenzofuran moiety, each substituent optionally containing at least one hetero atom, and R can be as shown for Structure 4. Substituents of the 4, 5, 6 and 7 carbon atoms in positions of the isobenzofuran moiety can be the same as the substituents of *meso* and/or of *β* positions of the porphyrin moiety, or substituents of the 4, 5, 6 and 7 carbon atoms in positions of the isobenzofuran moiety can differ from the substituents of *meso* and/or of *β* positions of the porphyrin moiety. Further, a substituent may be annelated to a carbon atom in *β* position and one carbon atom in *meso* position of the porphyrin moiety (schematically shown in Fig. 9a), and/or a substituent may be annelated to two neighbouring carbon atoms in *β* positions of the porphyrin moiety (schematically shown in Fig. 9b).

The compounds of the invention have the advantage of not agglomerating; especially not forming agglomerates by their porphyrin moieties. Currently, it is assumed that the isobenzofuran cycloaddition product prevents stacking of the porphyrin moieties.

In a preferred embodiment, the compound is for use as a medicament, e.g. for use in a medical treatment, e.g. for use in photodynamic therapy, especially for use in the treatment of pre-cancerous tissue, of solid cancer, e.g. skin cancer, prostate cancer, of a skin disease, e.g. psoriasis, acne, skin infections, macular degeneration, in antimicrobial photodynamic chemotherapy, especially for the treatment of oral infections and/or of biofilms, e.g. attached to tissue or mucosa, including infections by antibiotic-resistant microorganisms. The medicament comprising the compound can e.g. contain the compound in an aqueous or lipophilic composition, e.g. in a formulation for injection, especially for intra-tumoral injection, for systemic injection, or in a formulation for topic application. Substituents having dendrimer portions, e.g. as shown for R4 and R5, have the advantage of having low immunogenicity or being non-immunogenic.

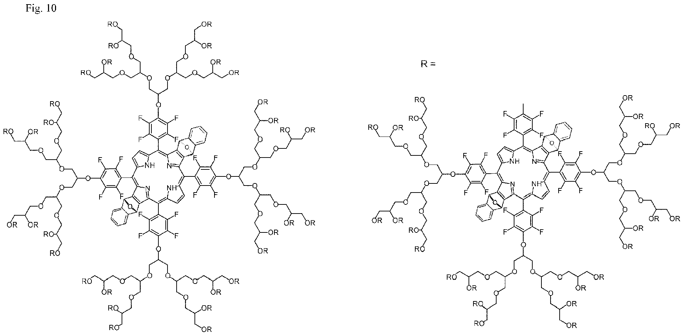

Optionally, the compounds of the invention can be contained in molecules which contain at least two or at least three of Structure 1 and/or Structure 2, e.g. linked together by their substituents. Such molecules may have a molecular weight in the range of 20 to 200 kDa, e.g. to provide for the EPR effect resulting in an accumulation of the molecules in tumour tissue after systemic administration. As an example, a compound having a molecular weight in this range can be obtained by reacting Structure 4 which is substituted with R5 in all *meso* positions, with additional Structure 4 IV which is substituted with R3, and subsequent dendronisation. An exemplary compound is shown in Fig. 10.

The compounds have the advantage of having an absorption maximum of 740 nm, which is in the range of wavelengths for which human and animal tissue is transmissible, e.g. to a depth of 1 to 4 cm. Accordingly, the compounds are suitable for use in photodynamic therapy, wherein the compounds are located within the tissue and the compounds are accessible by irradiation. Irradiation is by light comprising an activating wavelength, e.g. 700 to 800 nm, preferably comprising 740 nm, or consisting thereof. Irradiation of the compounds in the presence of oxygen, e.g. oxygen dissolved in living blood, mucus or tissue, or gaseous oxygen results in the production of singlet oxygen, optionally also other ROS, which are known to inactivate mammalian cells, bacteria and viruses. In the following, references to singlet oxygen comprise other ROS.

The compounds have the advantage of allowing various substituents, especially bound to the porphyrin moiety, so that the hydrophilicity, respectively lipophilicity, can be adjusted by the substituents.

Optionally, the substituents, especially substituents to the porphyrin moiety, can comprise a peptide that has affinity for cancer cells or for bacteria or viruses. In this embodiment, the peptide having affinity for cancer cells or for bacteria or for viruses promotes the arrangement of the compound in the vicinity of cancer cells, bacteria or viruses, respectively, and singlet oxygen is generated upon irradiation in this vicinity.

In a further embodiment, the compounds can be used in technical processes for producing singlet oxygen, e.g. for disinfection of air or water. The compounds can be immobilized on a carrier, e.g. adsorbed to a carrier, preferably covalently bound by a substituent to a carrier. In a process for producing singlet oxygen, the immobilized compound is contacted with oxygen and irradiated. Irradiation can generally be by light comprising an activating wavelength, e.g. sunlight, preferably light comprising 700 to 800 nm, preferably comprising 740 nm, or consisting thereof. For immobilizing the compound, the carrier can e.g. be selected from functionalized resins, e.g. weak ion exchange resins, e.g. resins having free amine groups. Preferably, the carrier is at least partially transmissible for light of the activating wavelength. Optionally, the compounds can be immobilized on a surface in order to provide production of singlet oxygen upon irradiation, e.g. for providing surfaces which upon irradiation are self-sterilizing.

In a further embodiment, the invention provides for novel compounds, e.g. for use as IR dye. In this embodiment, the compounds contain a metal ion, preferably Ni, complexed in the porphyrin moiety of Structure 1 and/or of Structure 2, e.g. complexed in the porphyrin moiety of Structure 3 and/or of Structure 4. Schematically, these compounds are 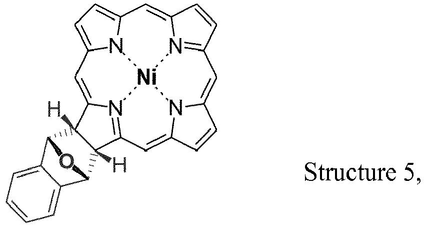 and/or preferably

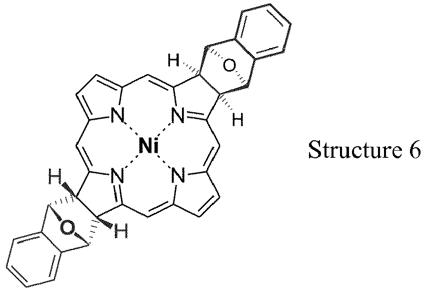

In a preferred embodiment, the compound comprises 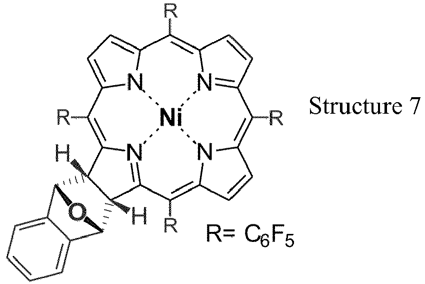 and/or preferably

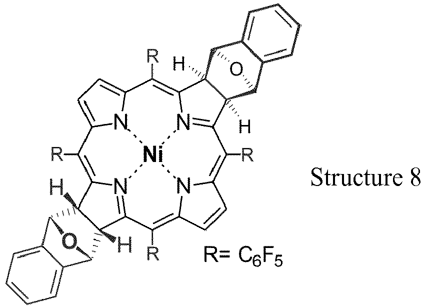

In this embodiment, the substituents R can alternatively be those described for Structure 3 and Structure 4.

Also the embodiment of the compounds containing a metal ion complexed within the porphyrin moiety has the advantages of being stable against oxidation and of being producible by a process for production which can be performed at temperatures below 100°C, e.g. at room temperature to 70°C, of using only fully synthetic starting compounds, i.e. no starting compounds isolated from a natural source, and of being free from metal-containing catalysts, e.g. free from OsO₄. Embodiments containing Ni as the metal ion complexed within the porphyrin moiety do not generate singlet oxygen. In embodiments containing complexed Ni²⁺, the compounds are not fluorescent. Embodiments containing a Pt ion or Pd ion as the complexed metal ion under irradiation show intense production of singlet oxygen.

In a further embodiment, the invention provides a process for producing compounds comprising Structure 1 and/or Structure 2, preferably Structure 3 and/or Structure 4. The process for producing the compounds comprises the step of performing a Diels-Alder-cycloaddition on a porphyrin moiety with IBF. In the process, the porphyrin moiety can optionally carry substituents, e.g. on carbon atoms which are in the *β* positions of the porphyrin. The process for producing the compounds has the advantage of process temperatures below 100°C, e.g. at a temperature between room temperature and 70°C, and that the process is free from metal-containing catalysts, e.g. free from OsO₄. Further, the starting compounds, i.e. the porphyrin-containing starting compound and isobenzofuran, are available as fully synthetic compounds also in a commercial scale, so that the process can be performed without isolation of natural compounds, e.g. without use of compounds isolated from natural sources. Isobenzofuran is e.g. available by the process according to Peters and Herges, Beilstein J. Org. Chem. 2017, 13, 2659-2662. Accordingly, the process preferably is performed completely synthetically, i.e. without use of a starting compound isolated from a natural source. The production processes have the advantage of having high yields.

The invention is also described with reference to the figures, which show in
- Fig. 1 a schematic representation of a synthetic process of the invention,
- Fig. 2 a schematic representation of a further synthetic process of the invention,
- Fig. 3 a schematic representation of a synthetic process of the invention,
- Fig. 4 a schematic representation of a further synthetic process of the invention,
- Fig. 5 a schematic representation of a synthetic process of the invention,
- Fig. 6 a schematic representation of a synthetic process of the invention for a metal-complex of the invention,
- Fig. 7 results for stability measurements of compounds of the invention and of comparative compounds, and the rate of singlet oxygen production measured as the decrease of the trapping reagent diphenylbenzisofuran as a function of irradiation time,
- Fig. 8 shows results of absorption and fluorescence measurements for compounds of the invention,
- Fig. 9 exemplary compounds of the invention having annelated substituents, and
- Fig. 10 an exemplary compound having a molecular weight in the range of 20 to 200 kDa.

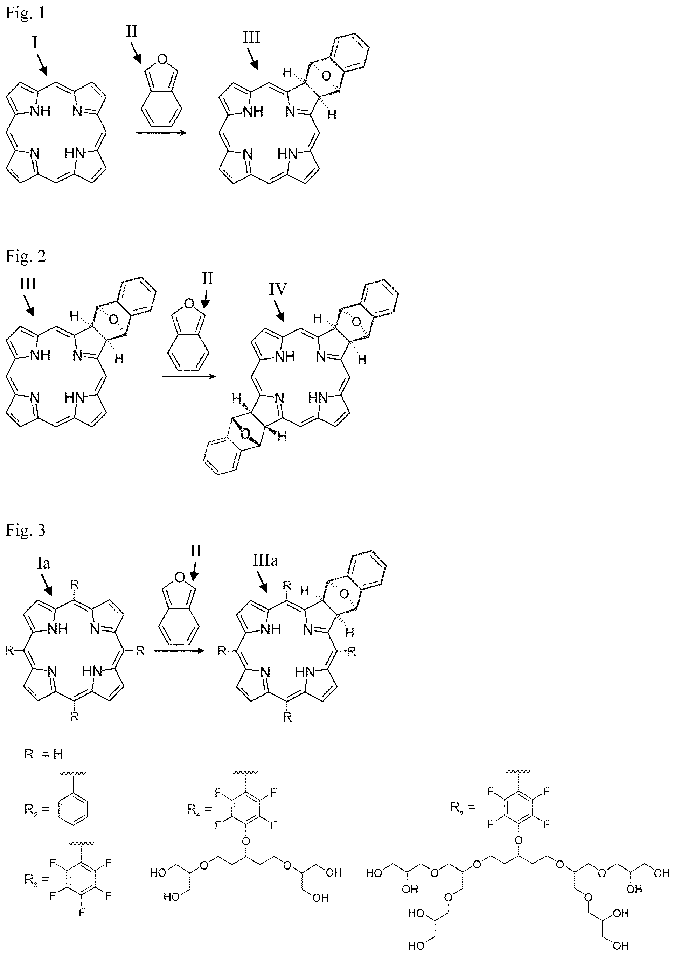

Schematically, the synthesis of a compound of Structure 1 by the Diels-Alder cycloaddition reaction is according to Fig. 1. As the porphyrin moiety, porphyrin I is reacted with isobenzofuran II to yield the chlorin III according to structure 1.

As shown in Fig. 2, the further reaction of the chlorin III with isobenzofuran II yields bacteriochlorin IV according to Structure 2.

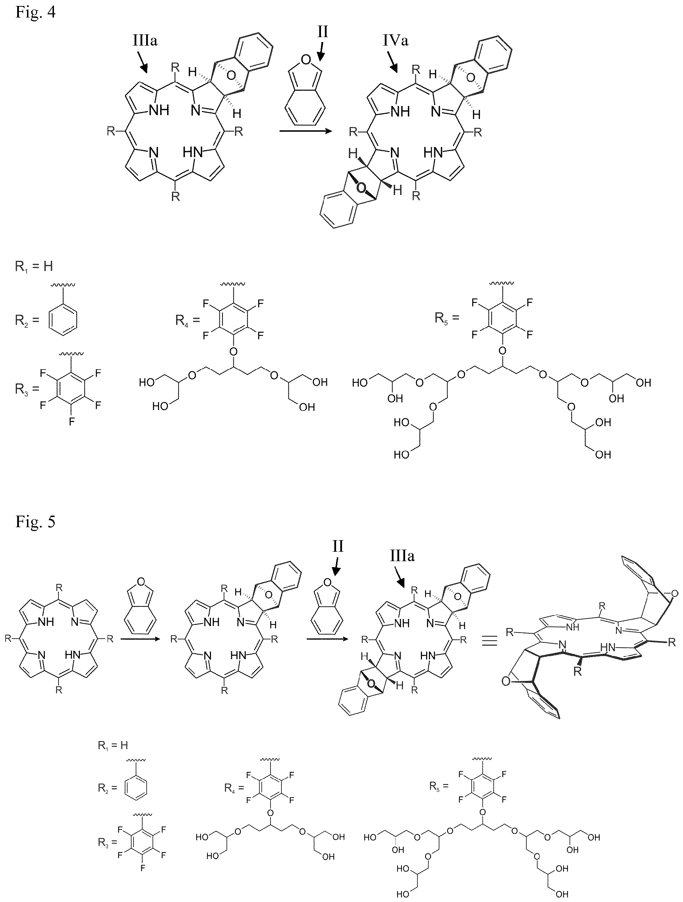

In Figures 3 and 4, exemplary substituents to the porphyrin moiety are shown. Other substituents, e.g. linear, branched or circular alkyl or aryl groups, e.g. C1 to C12, optionally containing at least one hetero atom, and aliphatic or aromatic groups, e.g. containing at least one five-membered or six-membered ring, optionally containing at least one hetero atom, e.g. halogens (e.g. F, Cl, Br) or functional groups such as ether, carbonyl and/or acid groups, can form substituents. Further optionally, at least one or all of the *meso* positions of the porphyrin can form a five- or six-membered ring to form a bridge with one of the neighbouring *β* positions. The *β* positions can also be substituted, either by alkyl, aryl, or heteroatom substituents, or two neighbouring *β* positions can be connected by a ring.

Fig. 3 shows that the process for producing a compound comprising Structure 1, e.g. to produce a chlorin IIIa, e.g. a compound of Structure 3, can also be done using a porphyrin Ia which is substituted, e.g. at those carbon atoms which are not part of the 5-membered rings of the porphyrin portion I, la, in a Diels-Alder cycloaddition with isobenzofuran II.

Fig. 4 shows that the process for producing a compound comprising Structure 2 can be done using a chlorin IIIa as a porphyrin moiety which is substituted, e.g. at those carbon atoms which are not part of the 5-membered rings of the prophyrin moiety, in a Diels-Alder-cycloaddition with isobenzofuran II to produce a substituted bacteriochlorin IVa, e.g. of Structure 4.

In the examples, isobenzofuran was synthesized according to Peters and Herges, Beilstein J. Org. Chem. 2017, 13, 2659.

### Example 1: Production of Structure 3

For synthesis of 5,10,15,20-tetrakis-(2,3,4,5,6-pentafluorophenyl)-chlorin according to Structure 3 having pentafluorophenyl as substituents R,
5,10,15,20-Tetrakis(pentafluorphenyl)porphyrin (50.0 mg, 51.3 µmol, (obtained from Sigma-Aldrich Company) was dissolved in 15 ml of benzene under a nitrogen atmosphere. Then a freshly prepared isobenzofuran solution in benzene (257 µmοl, 5 eq.) was added dropwise. After stirring for 36 h at 70°C, the solvent was removed under reduced pressure and the crude product was purified by column chromatography on silica gel (dichloromethane/pentane 7:3, Rf = 0.68). Green crystals were obtained. Yield: 49.3 mg (45.1 µmοl, 88%).

The structure of the product was determined as

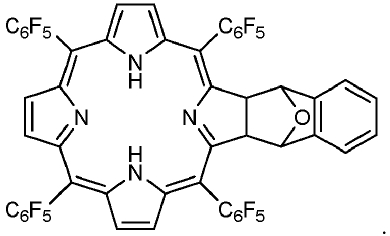

### Example 2: Production of Structure 4

For synthesis of 5,10,15,20-Tetrakis-(2,3,4,5,6-pentafluorophenyl)-bacteriochlorin according to Structure 4 (substituted with R2 = pentafluorophenyl), two processes could be employed:
Process 1: 5,10,15,20-Tetrakis(pentafluorophenyl)porphyrin (50.0 mg, 51.3 µmol) was dissolved in 15 ml of benzene under a nitrogen atmosphere. Then a freshly prepared isobenzofuran solution in benzene (770 µmol, 15 eq.) was added dropwise. After stirring for 36 h at 70°C, the solvent was removed under reduced pressure and the crude product was purified by column chromatography on silica gel (dichloromethane/pentane 7:3, Rf = 0.49). A green solid was obtained. Yield: 40.3 mg (33.3 µmοl, 65%).
Process 2: 5,10,15,20-Tetrakis-(2,3,4,5,6-pentafluorophenyl)-chlorin (50.0 mg, 45.8 µmol) was dissolved in 15 ml of benzene under a nitrogen atmosphere. Then a freshly prepared isobenzofuran solution in benzene (458 µmol, 10 eq.) was added dropwise. After stirring for 36 h at 70°C, the solvent was removed under reduced pressure and the crude product was purified by column chromatography on silica gel (dichloromethane/pentane 7:3, Rf = 0.49). A green solid was obtained. Yield: 45.5 mg (37.6 µmοl, 82%).

For both process 1 and process 2, the structure of the product was determined as

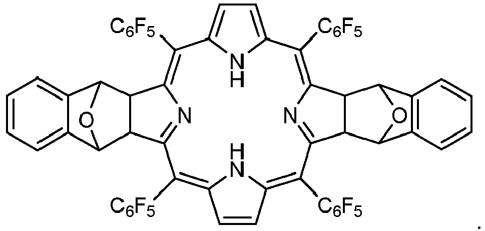

Fig. 5 schematically shows the synthetic routes of Example 1 and Example 2. Using X-ray analysis, it was found that the Diels-Alder reaction product contains the porphyrin and the substituents originating from the condensed isobenzofuran essentially completely in an anti - endo configuration as shown in Fig. 5.

### Example 3: Production of Structure 5

For synthesis of 5,10,15,20-Tetrakis-(2,3,4,5,6-pentafluorophenyl)-nickel(II)chlorin according to Structure 5 having pentafluorophenyl as substituents R,
5,10,15,20-Tetrakis(pentafluorophenyl)nickel(II)-porphyrin (50.0 mg, 48.5 µmol) was dissolved in 15 ml of benzene under a nitrogen atmosphere. Then a freshly prepared isobenzofuran solution in benzene (243 µmol, 5 eq.) was added dropwise. After stirring for 36 h at 70°C, the solvent was removed under reduced pressure and the crude product was purified by column chromatography on silica gel (dichloromethane/pentane 7:3, Rf = 0.68). A turquoise solid was obtained. Yield: 9.00 mg (6.97 µmοl, 14%). The structure of the product was determined as

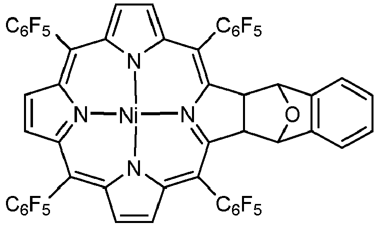

### Example 4: Production of Structure 6

For synthesis of 5,10,15,20-Tetrakis-(2,3,4,5,6-pentafluorophenyl)-nickel(II)-bacteriochlorin according to Structure 6 having pentafluorophenyl as substituents R,
5,10,15,20-Tetrakis-(2,3,4,5,6-pentafluorophenyl)-bacteriochlorin (50.0 mg, 41.3 µmol) and 18 eq. bis(cycloocta-1,5-dien)nickel (Ni(COD)₂) (205 mg, 744 µmol) were dissolved in 50 ml of toluene in the glove box. After 30 min of stirring at room temperature, the solvent was removed under reduced pressure and the crude product purified by column chromatography on silica gel (dichloromethane/pentane 1:1). A turquoise solid was obtained. Yield: 413.6 mg (10.7 µmol, 26%). The structure of the product was determined as

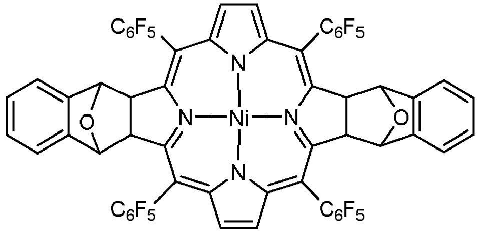

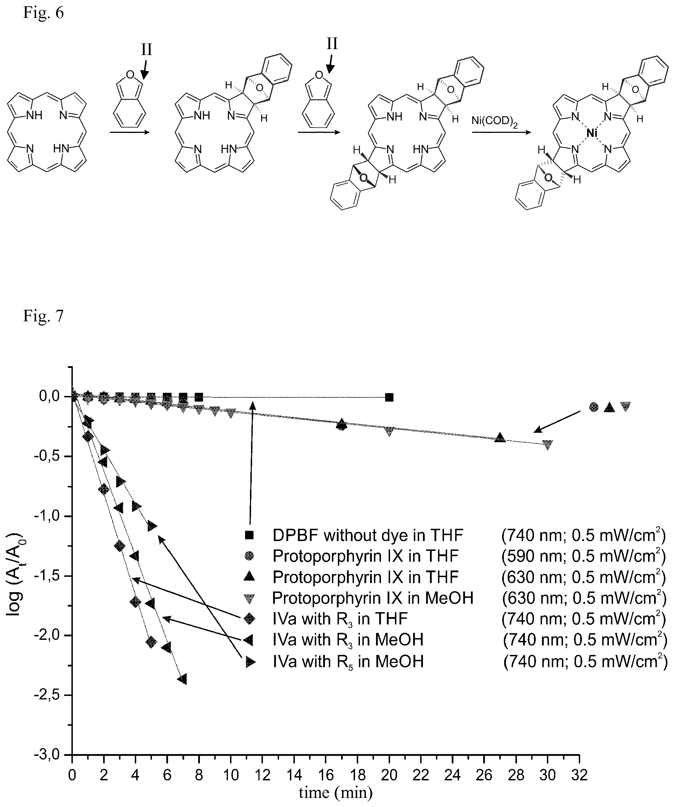

Fig. 6 schematically shows the synthetic routes of Example 3 and Example 4. Using X-ray analysis, it was found that the Diels-Alder reaction product contains the porphyrin and the substituents originating from the condensed isobenzofuran in an anti - endo configuration as shown.

### Example 5: Production of Structure 4 substituted in all meso positions

For synthesis of acetal protected *bacteriochlorin,* sodium hydride (21.8 mg, 544 µmol) (60% dispersion in mineral oil) was suspended in 7 mL of dry tetrahydrofuran under nitrogen atmosphere. [G2.0]-OH (248 mg, 496 mol, this second generation glycerol (G[2.0]-OH) was synthesised according to a procedure of Haag et al. as described in: M. Wyszogrodzka and R. Haag, Chem. Eur. J., 2008, 14, 9202-9214) was dissolved in 6 mL of tetrahydrofuran and slowly added to the sodium hydride suspension. The suspension was stirred for 30 min. *Bacteriochlorin* (75.0 mg, 62.0 µmol) was added and the mixture was stirred for 4 d. The reaction was quenched with water and added to 100 mL of diethyl ether. The organic layer was washed with 100 mL of 0.1 M hydrochloric acid and dried over magnesium sulphate. The solvent was removed under reduced pressure. The crude product was purified by column chromatography (silica gel, dichloromethane/methanol = 97/3, *R*f = 0.49). The product was obtained as a green, viscous oil.
Yield: 128 mg (34.8 µmοl, 56%). The following structure was determined:

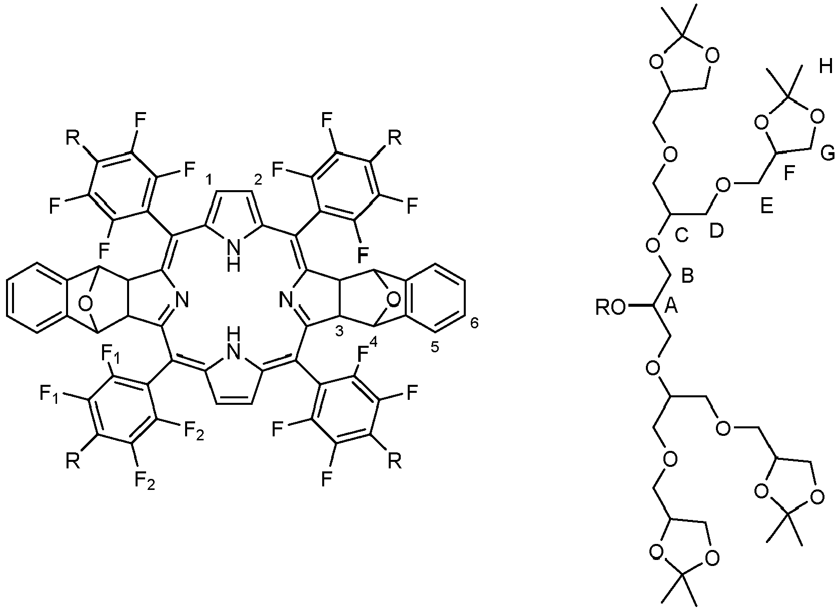

For synthesis of the deprotected *bacteriochlorin (5),*
acetal protected porphyrin (38.0 mg, 10.3 µmοl) was dissolved in a mixture of 0.30 mL of acetic acid, 1 mL of methanol and 0.50 mL of water and stirred for 18 h at 40 °C. The solvent was removed under reduced pressure. The product was obtained as a green, viscous oil. Yield: 30.0 mg (9.97 µmοl, 97%). The following structure was determined 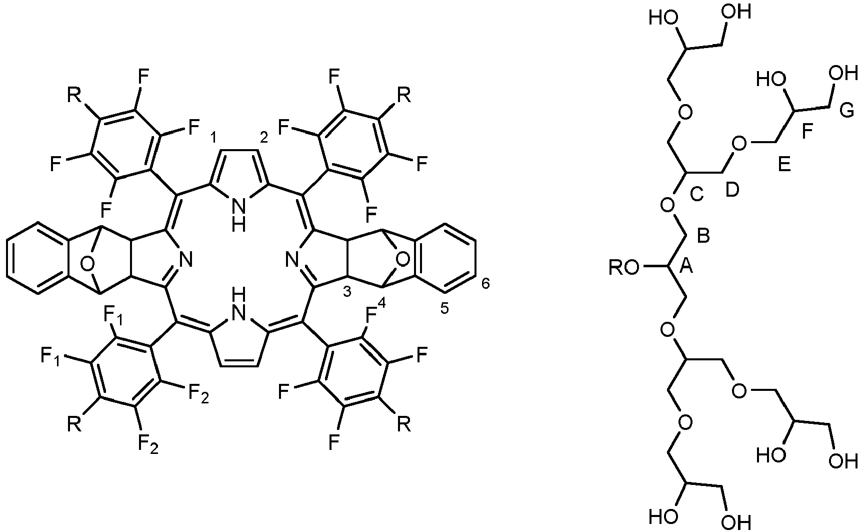 , wherein in the substituent R, the designators A, B, C, D, E, F and G indicate the carbon atoms of the dendrimer chain.

### Example 6: Production of singlet oxygen under irradiation

As a representative for a compound comprising Structure 2, the 5,10,15,20-Tetrakis-(2,3,4,5,6-pentafluorophenyl)-bacteriochlorin was dissolved in a concentration of 5 µM and irradiated with light of 740 nm, 0.5 mW/cm² in the presence of 1,3-diphenylisobenzofuran (DPBF) as an indicator for presence of singlet oxygen. The decrease of the absorbance band at 410 nm was monitored as a measure for oxidation of the DPBF by the reactive singlet oxygen.

Fig. 7 shows the results of the decrease of the absorbance band of DPBF, in comparison to DPBF in tetrahydrofuran (THF) irradiated with 740 nm, of protoporphyrin IX in THF irradiated with 590 nm, of protoporphyrin IX (obtained from ABCR Germany) in THF irradiated with 630 nm, of protoporphyrin IX in methanol irradiated with 630 nm, and of 5,10,15,20-tetrakis-(2,3,4,5,6-pentafluorophenyl)-bacteriochlorin according to Structure 2 (3 in Fig. 7) in THF or in methanol, and of 5,10,15,20-tetrakis-(2,3,4,5,6-pentafluorophenyl)-bacteriochlorin according to Structure 2 substituted with R5 = tetrafluorophenyl substituted in *para* position with a dendrimer (5 in Fig. 7) in methanol, irradiated with 740 nm. Measurements of singlet oxygen production by Structure 2 substituted with R5 were also made using betanin, which is water-soluble, as an indicator. Structure 2 substituted with R5 is soluble in water and aqueous media, e.g. blood serum.

These results show that the indicator DPBF under irradiation, without a sensitizer added, is essentially stable and that the protoporphyrin IX effectively produces singlet oxygen. The compounds of the invention comprising Structure 2 show a markedly higher production of singlet oxygen, for Structure 2 substituted with pentafluorophenyl a singlet oxygen production that is 30 times higher in THF compared to protoporphyrin IX, and for Structure 2 substituted with R5 a singlet oxygen production that is 16 times higher. Further, the constant rates of singlet oxygen production show that the compounds of the invention are stable against degradation under conditions of singlet oxygen production.

### Example 7: Photostability measurements

The stability of compounds of the invention against irradiation was measured by irradiating 5,10,15,20-tetrakis-(2,3,4,5,6-pentafluorophenyl)-bacteriochlorin according to Structure 2 and 5,10,15,20-tetrakis-(2,3,4,5,6-pentafluorophenyl)-bacteriochlorin according to Structure 2 substituted with R5 at 740 nm with an intensity of 1 W/cm² for 60 min. For determination of degradation (photobleaching), the absorbance maxima at 733 nm were determined. Both compounds showed high stability by showing no decomposition and no change in the UV spectrum.

### Example 8: Absorption and fluorescence measurements

The optical properties of compounds of the invention were measured for the examples of 5,10,15,20-tetrakis-(2,3,4,5,6-pentafluorophenyl)-bacteriochlorin according to Structure 2 and 5,10,15,20-tetrakis-(2,3,4,5,6-pentafluorophenyl)-bacteriochlorin according to Structure 2 substituted with R5. The compounds were dissolved in methanol to 10 µM, measurements were made at 25°C. Fig. 8 shows the absorbance (solid line) and the fluorescence (dashed line), in Fig. 8 A) for 5,10,15,20-tetrakis-(2,3,4,5,6-pentafluorophenyl)-bacteriochlorin, in Fig. 8 B) for 5,10,15,20-tetrakis-(2,3,4,5,6-pentafluorophenyl)-bacteriochlorin according to Structure 2 substituted with R5. Both compounds show excellent optical properties in solution, e.g. a sharp absorption in the near infrared region at 733 nm and with moderate quantum yields of fluorescence at 738 nm.

## Claims

1. Compound comprising

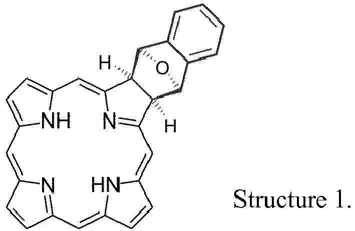

2. Compound according to claim 1, comprising

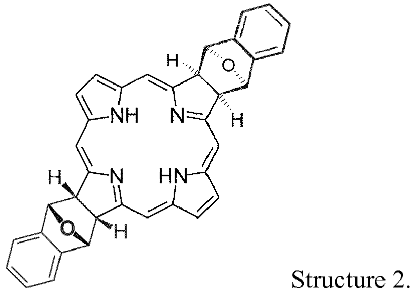

3. Compound according to one of the preceding claims, wherein the structure comprises substituents according to 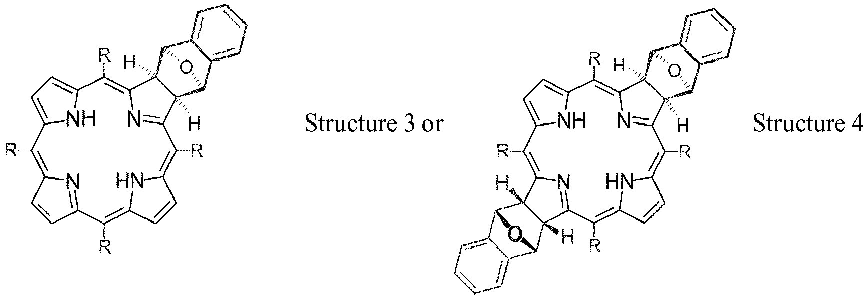 wherein at least one substituent R in the *meso* position of the porphyrin and/or at least one substituent in the *β* position of the porphyrin is independently selected from hydrogen, linear, branched or circular alkyl or aryl groups, e.g. C1 to C12, optionally containing at least one hetero atom and/or a functional group such as ether, carbonyl and/or acid groups, and aliphatic or aromatic groups, e.g. containing at least one five-membered or six-membered ring, optionally containing at least one hetero atom, e.g. , or R₁ = H 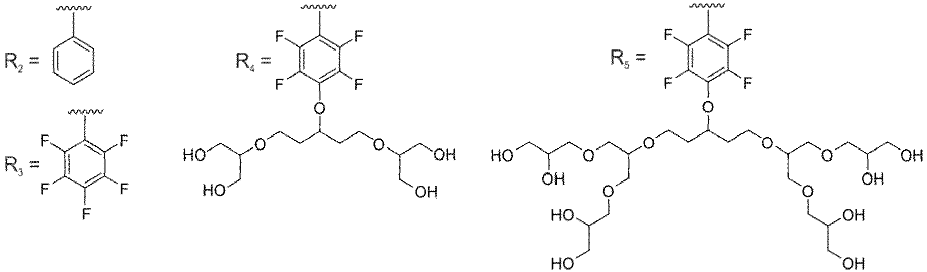 and/or two neighbouring *β* positions or one *β* position and one *meso* position are connected by a five- or six-membered ring forming an annelated substituent connecting two neighbouring *β* positions or one *β* position and one *meso* position, or one, two, three or all four *meso* positions each are connected to a neighbouring β position by an annelated substituent.

4. Compound according to one of the preceding claims, wherein the compound is immobilized on a carrier.

5. Compound according to one of the preceding claims, containing a metal ion complexed within the porphyrin moiety.

6. Compound according to one of the preceding claims, wherein at least one of the carbon atoms of the phenyl ring of the isobenzofuran moiety is substituted by a substituent which is independently selected from hydrogen, linear, branched or circular alkyl or aryl groups, e.g. C1 to C12, optionally containing at least one hetero atom and/or a functional group such as ether, carbonyl and/or acid groups, and aliphatic or aromatic groups, e.g. containing at least one five-membered or six-membered ring, optionally containing at least one hetero atom, or R₁ = H

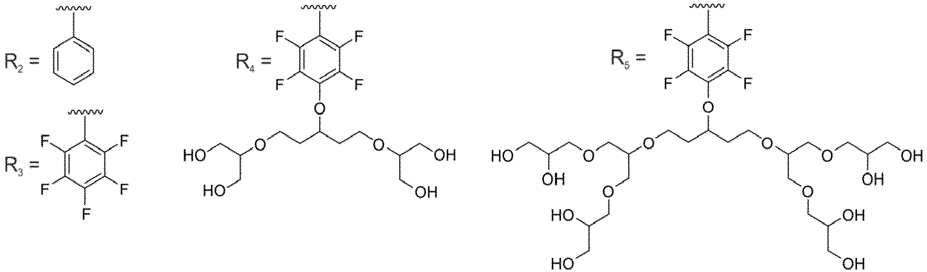

7. Compound according to one of the preceding claims for use in medical therapy.

8. Compound according to claim 7 for use in medical therapy, wherein the medical therapy is a photodynamic therapy for the treatment of solid tumours, of a skin disease, an infection, e.g. a bacterial, fungal or viral infection, of macular degeneration, wherein the infectious agent may be resistant to at least one antibiotic.

9. Compound according to one of claims 7 to 8 for use in medical therapy of solid tumours, **characterized in that** the compound is contained in a molecule having a size in the range of 20 to 200 kDa.

10. Process for producing a compound according to one of claims 1 to 7, **characterized by** comprising a Diels-Alder cycloaddition reaction condensing a porphyrin moiety with one or two isobenzofuran per porphyrin moiety.

11. Process according to claim 10, **characterized in that** the Diels-Alder cycloaddition reaction is carried out at a temperature below 100°C.

12. Process according to one of claims 10 to 11, **characterized in that** the Diels-Alder cycloaddition reaction is carried out in the absence of a metal-containing catalyst.

13. Process according to one of claims 10 to 12, **characterized in that** the porphyrin is not isolated from a natural source.

14. Process for producing singlet oxygen from oxygen by irradiating a compound which is in contact with oxygen, **characterized in that** the compound is according to one of claims 1 to 10.

15. Process according to claim 14, **characterized in that** the compound is immobilized on a carrier and is in contact with an aqueous or gaseous medium containing oxygen while the compound is irradiated.
